# DEMANDE DE BREVET EUROPEEN

(11) **EP 2 251 004 A2**
(43) Date de publication de la demande: **17.11.2010**
(21) Numéro de dépôt: 10171442.6
(22) Date de dépôt: 09.11.2006
(51) Int. Cl.: A61K 9/107, A61P 17/06, A61K 31/59, A61K 31/203, A61K 8/06, A61Q 19/00, A61K 31/573

(54) **Composition pharmaceutique ou cosmétique, et procédé de solubilisation mixte pour préparer la composition**

(30) Priorité: 10.11.2005 FR 0511448
(62) Demande divisionnaire de: 06842010.8
(71) Demandeur: Galderma Research & Development, 06410 Biot (FR)
(72) Inventeur: Brzokewicz, Alain, 06560, VALBONNE (FR)
(74) Mandataire: Allab, Myriam

(57) **Abrégé**

L'invention concerne une composition pharmaceutique ou cosmétique comprenant au moins une phase hydrophile, au moins une phase lipophile, et au moins un principe actif, ledit principe actif étant présent sous forme solubilisée dans chacune de ces phases, ainsi que le procédé de fabrication de cette composition.

## Description

La présente invention concerne une composition à usage pharmaceutique ou cosmétique, comprenant au moins deux phases dans lesquelles est solubilisé un même principe actif, ainsi que le procédé de fabrication de cette composition.

Il est commun de fabriquer et d'utiliser en pharmacie ou en cosmétique des émulsions dans lesquelles le principe actif est solubilisé dans une seule phase, que ce soit une phase hydrophile ou une phase lipophile.

Or, la solubilisation d'un principe actif dans une seule phase n'est pas exempte de problèmes. Effectivement, la concentration maximale du principe actif, solubilisable dans une phase, peut être inférieure à la concentration thérapeutiquement efficace dans la ou les pathologies visées.

Des phénomènes de migration du principe actif de sa phase solvante vers l'autre phase peuvent en outre apparaître et entraîner sa recristallisation. Dans ce cas, la recristallisation peut s'avérer particulièrement problématique lorsque le principe actif a un potentiel irritant. Des problèmes d'irritation peuvent être rencontrés, dus au contact direct des cristaux avec la peau, lors de l'application de la formulation.

Ces mêmes problèmes de recristallisation peuvent se manifester lorsque le principe actif est dispersé dans une phase très faiblement solvante de ce principe actif.

On entend par actif sous forme dispersée, un principe actif sous forme de particules solides, mises en suspension dans un véhicule donné. De façon préférentielle, les particules dispersées sont visibles à l'oeil nu ou en microscopie optique telle que la microscopie en polarisation croisée. Ainsi, la taille des particules dispersées est préférentiellement supérieure ou égale à 1 micron.

Pour améliorer la pénétration cutanée, le brevet US 6,491,396 propose une émulsion huile-dans-eau (H/E) au sein de laquelle le principe actif, le maxacalcitol, dérivé de la vitamine D3, est présent dans la phase lipophile et/ou dans la phase hydrophile. Le maxacalcitol est alors dans un état solubilisé dans la phase lipophile, et dans un état dispersé dans la phase hydrophile.

Une telle émulsion n'est pas entièrement satisfaisante. En particulier, les inconvénients liés à la dispersion dans un véhicule sont multiples. Ainsi, au-delà d'une certaine concentration, des problèmes d'agrégats et d'hétérogénéité de la dispersion peuvent apparaître. La recherche d'un agent dispersant adapté au principe actif n'est en outre pas toujours évidente. La granulométrie du principe actif doit également faire l'objet de beaucoup d'attention en fonction du site d'application et de la cible biologique visée.

D'autre part, la présence dans la formulation du principe actif dans deux états différents (solubilisé dans une phase et dispersé dans l'autre) peut s'avérer préjudiciable. En effet, le principe actif dispersé constitue un germe de recristallisation et peut entraîner un problème de croissance cristalline, phénomène difficilement contrôlable et néfaste pour la formulation en terme de stabilité physique et d'efficacité thérapeutique.

Les inventeurs ont maintenant mis en évidence qu'il était possible de résoudre les problèmes évoqués ci-dessus, par une solubilisation du principe actif dans les deux phases de la composition En particulier, ils ont montré que de manière surprenante, bien que le principe actif soit solubilisé dans les deux phases, la composition est stable physiquement et permet d'atteindre une concentration globale plus élevée du principe actif thérapeutiquement efficace.

Par "stabilité physique" de la composition selon l'invention, on entend notamment une composition qui ne présente pas de déphasage au cours du temps après stockage pendant trois mois à 4°C, à température ambiante et à 40°C. Selon l'invention, un autre paramètre de stabilité physique observé est notamment l'absence de phénomène de recristallisation à température ambiante et à 4°C, pendant trois mois.

En effet, à la concentration thérapeutiquement efficace, aucun phénomène de recristallisation du principe actif n'a été observé, après stockage des formulations pendant 3 mois à température ambiante et à 4°C.

Dans l'ensemble du présent texte, on entend par principe actif sous forme solubilisée, une dispersion à l'état moléculaire dans un liquide, aucune cristallisation de l'actif n'étant visible à l'oeil nu ni même au microscope optique en polarisation croisée, ou à l'aide d'autres techniques plus précises telle que la microscopie électronique. De préférence, les particules de principe actif ont une taille inférieure à 1 nm.

Préférentiellement selon l'invention, on entend par principe actif sous forme solubilisée, une dispersion à l'état moléculaire dans un liquide pour laquelle aucune cristallisation de l'actif n'est visible à l'oeil nu ou au microscope optique.

La présente invention concerne donc une composition pharmaceutique ou cosmétique comprenant au moins deux phases de polarités distinctes, un même principe actif présent sous forme solubilisée dans chacune de ces phases, ainsi que le procédé de fabrication de cette composition.

L'invention a donc pour objet une composition pharmaceutique ou cosmétique comprenant au moins une phase hydrophile, au moins une phase lipophile, et au moins un principe actif, ledit principe actif étant présent sous forme solubilisée dans au moins ladite phase hydrophile et au moins ladite phase lipophile.

De préférence la composition est une émulsion.

De préférence, la composition comprend une phase hydrophile (et une seule) et une phase lipophile (et une seule).

Conformément à l'invention, la solubilisation du principe actif dans les deux phases de la composition offre de multiples avantages.

Elle peut permettre de moduler la libération du principe actif. Par exemple, dans une émulsion H/E (huile dans eau), la fraction du principe actif qui est solubilisée dans la phase externe de l'émulsion (ici la phase hydrophile), est libérée avant la fraction solubilisée dans la phase interne (ici la phase lipophile). Cette libération différée du principe actif confère ainsi à la formulation un rôle de réservoir du principe actif.

La solubilisation dans les différentes phases permet par ailleurs de diminuer les problèmes d'irritation, lorsque le principe actif est potentiellement irritant. D'une part le phénomène de recristallisation du principe actif est considérablement réduit, d'autre part la modulation de sa libération permet d'améliorer sensiblement la tolérance d'une formulation à usage topique en évitant une accumulation rapide du principe actif au niveau des assises cutanées, responsable des problèmes d'irritation. La solubilisation du principe actif dans les deux phases de la composition permet d'atteindre une concentration en principe actif supérieure à celle obtenue si celui-ci était solubilisé uniquement dans une seule phase. Cette concentration en principe actif solubilisé dans une seule phase s'avère très souvent inférieure à la concentration thérapeutiquement efficace. La solubilisation du principe actif dans les différentes phases de la composition permet d'atteindre une concentration suffisante pour obtenir une efficacité avérée dans la ou les pathologies visées.

### Les principes actifs :

Tout principe actif peut être utilisé, étant entendu qu'il doit être soluble et se trouver dans le même état moléculaire dans l'une et l'autre des phases de l'émulsion. En effet, il est connu que certains principes actifs peuvent être présents à l'état solubilisé dans les différentes phases d'une émulsion, mais sous forme salifiée dans l'une, et sous forme non salifiée dans l'autre. Or, le principe actif solubilisé dans les deux phases de la composition selon l'invention est présent dans le même état moléculaire.

De plus, dans la composition selon l'invention, le principe actif est présent dans le même état physique dans les deux phases de la composition. En effet, selon l'invention, le principe actif est solubilisé dans chacune des phases de la composition et en aucun cas dispersé dans l'une ou l'autre des 2 phases.

Les molécules utilisées en tant que principe actif dans ces compositions présentent donc de préférence un profil de solubilité particulier décrit ci-dessous.

Elles possèdent une solubilité suffisante dans l'eau ou les composés polaires classiquement utilisés dans le domaine pharmaceutique ou cosmétique, de préférence dans les formulations topiques.

Cette solubilité dans les milieux polaires permet leur solubilisation dans la phase hydrophile de la composition.

D'autre part, ces molécules possèdent une solubilité suffisante dans les excipients lipophiles habituellement utilisés dans le domaine pharmaceutique ou cosmétique, de préférence dans les formulations pour usage topique, permettant leur solubilisation dans la phase lipophile de l'émulsion.

Par solubilité suffisante, on entend une solubilité comprise entre 0.005% et 1%, préférentiellement entre 0.01% et 0.5% en poids d'actif par rapport au poids total du solvant (eau, composés polaires, excipients lipophiles).

Par « soluble », on entend la caractéristique de certains corps solides à former des solutions lorsqu'on les introduit dans un solvant approprié. Différents paramètres peuvent intervenir sur la solubilité d'une molécule dans le solvant considéré. Ainsi, le poids moléculaire, le point de fusion, la pureté chromatographique, la structure cristalline, mais aussi la structure chimique, et notamment la nature des groupements fonctionnels et des parties hydrophiles ou lipophiles, contribuent à son profil de solubilité.

Les paramètres permettant de caractériser le profil de solubilité d'une molécule sont notamment le paramètre de solubilité et le log P de la molécule.

Le paramètre de solubilité représente l'ensemble des interactions moléculaires solvant / soluté, à savoir les forces de dispersion, les interactions polaires et interaction de type liaison hydrogène.

La méthode de Hansen, connue de l'homme de l'art, permet de déterminer le paramètre de solubilité global d'une molécule de manière théorique (utilisation de la méthode de contribution de groupe appliquée à la molécule) et de manière expérimentale, par l'évaluation de la solubilité de cette molécule dans des solvants dont le paramètre de solubilité global a été déterminé.

L'intérêt de cette méthode est d'établir une liste de solvants potentiels pour la molécule étudiée.

Ces solvants potentiels ont un paramètre de solubilité proche de celui de la molécule.

Le paramètre de solubilité global des molécules utilisées dans les compositions de l'invention se situe entre 10 et 40, de préférence entre 15 et 30 et plus préférentiellement entre 18 et 24.

Un autre paramètre permettant d'évaluer la solubilité d'une molécule est son log P. Le log P d'une molécule est représenté par le rapport de sa solubilité dans l'octanol et de sa solubilité dans l'eau.

Ce paramètre est important pour les molécules utilisées en voie topique.

En effet ce partage octanol / eau est représentatif du partage de la molécule lors de son application sur la peau, partage entre les parties hydrophiles (cellules) d'une part et lipophiles (ciment intercellulaire) de la peau d'autre part.

Le logarithme du coefficient de partage (noté log P) permet alors de connaître sa polarité. En effet, plus la valeur de log P est grande, plus la molécule aura un caractère lipophile. Inversement plus cette valeur est faible, plus la molécule aura un caractère polaire. Les molécules utilisables dans la présente invention possèdent un log P compris entre 2 et 6, et préférentiellement entre 4 et 5, de manière à pouvoir être solubilisées dans la phase hydrophile, comme dans la phase lipophile de la composition.

En outre, les molécules utilisées en tant que principe actif sont de préférence chimiquement stables dans chacune des phases dans lesquelles elles sont solubilisées.

Les agents anti-inflammatoires stéroïdiens, les agents antifongiques, les antiparasitaires, les modulateurs des récepteurs nucléaires et les hormones stéroïdes sexuelles sont des exemples non exhaustifs de molécules possédant de telles propriétés physico-chimiques.

Parmi les agents anti-inflammatoires stéroïdiens, on peut citer principalement les corticostéroïdes comme par exemple la béclométhasone, la bétaméthasone, le clobetasol, la cortisone, le desonide, la dexaméthasone, le fluocloronide, le fluocinolonacetonide, le fluocinonide, le fludroxycortide, le formocortal, l'halcinonide, l'hydrocortisone, le méthylprednisolone, la prednisone, la prednisone, le triamcinolone, le triamcinolonacetonide ou leurs mélanges.

Parmi les agents antifongiques, on peut citer principalement l'amorolfine, le clotrimazole, le fluconazole, le ketoconazole, le miconazole ou encore le tolnaftate.

Parmi les agents antiparasitaires, on peut citer principalement l'ivermectine et plus généralement la famille des avermectines.

Les hormones stéroïdes sexuelles utilisables dans l'invention incluent la DHEA ou déhydroépiandrostérone, ainsi que ses précurseurs et dérivés chimiques et/ou biologiques.

Parmi les agents modulateurs des récepteurs nucléaires, on peut citer par exemple les rétinoïdes et leurs analogues, les composés modulateurs des récepteurs PPAR (Peroxisome-Proliferator Activated Receptor) et la vitamine D et ses analogues ou dérivés.

Parmi les rétinoïdes, on peut citer par exemple l'acide rétinoïque ou trétinoïne, l'acide 13-cis-rétinoïque ou isotrétinoïne, le rétinol, l'acitrétine, l'adapalène, ou leurs mélanges.

Parmi les composés modulateurs des récepteurs PPAR, on peut citer notamment les thiazolidinediones et dérivés.

Par analogues de vitamine D, on entend les différentes formes de vitamine D telles que par exemple la vitamine D₂ ou la vitamine D₃. Par « dérivés de vitamine D », on entend des composés qui présentent des propriétés biologiques analogues à celles de la vitamine D, notamment des propriétés de transactivation des éléments de réponse à la vitamine D (VDRE), telles qu'une activité agoniste ou antagoniste vis-à-vis de récepteurs de la vitamine D ou de ses dérivés. Ces composés ne sont généralement pas des métabolites naturels de la vitamine D. Il s'agit en particulier de composés synthétiques comprenant le squelette de la vitamine D avec des modifications sur les chaînes latérales et/ou comprenant également des modifications dans le squelette lui-même. Des composés dérivés de la vitamine D utiles selon l'invention comprennent ainsi des analogues structuraux, par exemple biaromatiques.

A titre illustratif de ces dérivés de vitamine D, on peut citer en particulier le calcipotriol, le calcitriol ou 1,25 dihydroxyvitamine D₃, le doxercalciférol, le sécalcitol, le maxacalcitol, le séocalcitol, le tacalcitol, le paricalcitol, le falécalcitriole, le 1α,24S-dihydroxy-vitamine D2, le 1(S),3(R)-dihydroxy-20(R)-[((3-(2-hydroxy-2-propyl)-phényl)-méthoxy)-méthyl]-9,10-sécopregna-5(Z),7(E),10(19)-triène, leurs mélanges et leurs dérivés.

Selon un mode de réalisation particulier, les dérivés de vitamine D utilisés de préférence selon l'invention sont décrits dans la demande WO 00/26167. Il s'agit de composés analogues structuraux de la vitamine D qui montrent une activité sélective sur la prolifération et sur la différenciation cellulaire sans présenter de caractère hypercalcémiant.

Ces composés peuvent être représentés par la formule générale (I) suivante : 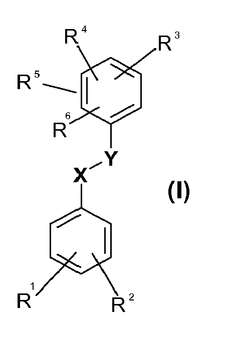 dans laquelle :
- R¹ représente un atome d'hydrogène, un radical méthyle ou un radical -(CH₂)ₙ-OR⁷,
- R² représente un radical -(CH₂)ₙ-OR⁸,
   n, R⁷ et R⁸ ayant les significations données ci-après,
- X-Y représente une liaison choisie parmi les liaisons de formules (a) à (d) suivantes pouvant être lues de gauche à droite ou inversement : 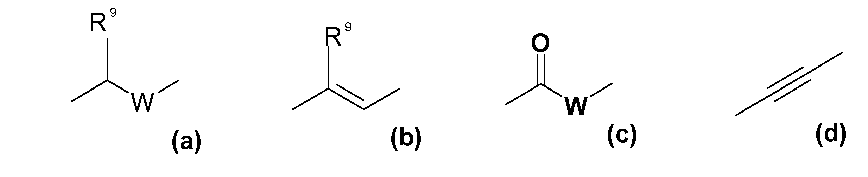 R⁹ et W ayant les significations données ci-après,
- R³ représente la chaîne de la vitamine D₂ ou de la Vitamine D₃, 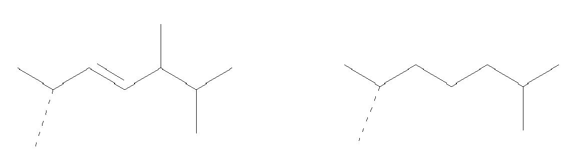 les traits en pointillés représentent la liaison reliant la chaîne au cycle benzénique représenté sur la figure (I), ou
- R³ représente une chaîne ayant de 4 à 8 atomes de carbone substituée par un ou plusieurs groupements hydroxyles, les groupements hydroxyles pouvant être protégés sous forme d'acétoxy, de méthoxy ou d'éthoxy, de triméthylsilyloxy, de tertiobutyldiméthylsilyloxy, de tétrahydropyranyloxy et éventuellement en outre :
- substituée par un ou plusieurs groupements alkyles inférieurs ou cycloalkyles et/ou
- substituée par un ou plusieurs atomes d'halogène, et/ou
- substituée par un ou plusieurs groupements CF₃, et/ou
- dans laquelle un ou plusieurs atomes de carbone de la chaîne sont remplacés par un ou plusieurs atomes d'oxygène, de soufre ou d'azote, les atomes d'azote pouvant éventuellement être substitués par des radicaux alkyles inférieurs, et/ou
- dans laquelle une ou plusieurs liaisons simples de la chaîne sont remplacées par une ou plusieurs liaisons doubles et/ou triples,
- R³ étant positionné sur le cycle benzénique en *para* ou *méta* de la liaison X-Y,
- R⁴, R⁵ et R⁶, identiques ou différents, représentent un atome d'hydrogène, un radical alkyle inférieur, un atome d'halogène, un radical -OR¹⁰, un radical polyéther,
   R¹⁰ ayant la signification donnée ci-après,
- n étant 0,1 ou 2,
- R⁷ et R⁸ identiques ou différents, représentent un atome d'hydrogène, un radical acétyle, un radical triméthylsilyle, un radical tertiobutyldiméthylsilyle, un radical tétrahydropyranyle,
- R⁹ représente un atome d'hydrogène ou un radical alkyle inférieur,
- W représente un atome d'oxygène, de soufre, un radical -CH₂- ou un radical

- NH- pouvant éventuellement être substitué par un radical alkyle inférieur,
- R¹⁰ représente un atome d'hydrogène ou un radical alkyle inférieur, ainsi que les isomères optiques et géométriques desdits composés de formule (I) ainsi que leurs sels dans le cas où X-Y représentent une liaison de formule (a) et W représente un radical -NH- éventuellement substitué par un radical alkyle inférieur.

Par l'expression « radical alkyle inférieur », on entend un radical alkyle linéaire ou ramifié ayant de 1 à 6 atomes de carbone.

Parmi les composés de formule (I) pouvant être utilisés dans les compositions de la présente invention, on peut notamment citer les suivants :
1. 6-[3-(3,4-bis-hydroxyméthyl-benzyloxy)-phényl]-2-méthyl-hepta-3,5-dien-2-ol,
2. 7-[3-(3,4-bis-hydroxyméthyl-phénoxyméthyl)-phényl]-3-éthyl-octan-3-ol,
3. 7-{3-[2-(3,4-Bis-hydroxyméthyl-phényl)-éthyl]-phényl}-3-éthyl-octa-4,6-dien-3-ol,
4. 6-{3-[2-(3,4-Bis-hydroxyméthyl-phényl)-éthyl]-phényl}-2-méthyl-hepta-3,5-dien-2-ol,
5. 7-{3-[2-(3,4-Bis-hydroxyméthyl-phényl)-vinyl]-phényl}-3-éthyl-octa-4,6-dien-3-ol,
6. 7-[3-(3,4-Bis-hydroxyméthyl-benzyloxy)phényl]-3-éthyl-3-octanol,
7. 4E,6E)-7-[3-(3,4-Bis-hydroxyméthyl-benzyloxy)-phényl]-3-éthyl-octa-4,6-dien-3-ol,
8. (4E,6E)-7-[3-(3,4-Bis-hydroxyméthyl-benzyloxy)-phényl]-3-éthyl-nona-4,6-dien-3-ol,
9. (E)-7-[3-(3,4-Bis-hydroxyméthyl-benzyloxy)-phényl]-3-éthyl-oct-4-en-3-ol,
10. (E)-7-[3-(3,4-Bis-hydroxyméthyl-benzyloxy)-phényl]-3-éthyl-oct-6-en-3-ol,
11. (E)-7-[3-(3,4-Bis-hydroxyméthyl-benzyloxy)-phényl]-3-éthyl-oct-6-en-4-yn-3-ol,
12. (4E,6E)-7-[3-(3,4-Bis-hydroxyméthyl-phénoxyméthyl)-phényl]-3-éthyl-octa-4,6-dien-3-ol,
13. (E)-7-[3-(3,4-Bis-hydroxyméthyl-phénoxyméthyl)-phényl]-3-éthyl-non-6-en-3-ol,
14. (E)-7-{3-[(3,4-Bis-hydroxyméthyl-benzyl)-méthylamino]-phényl}-3-éthyl-oct-6-en-3-ol, et
15.7-[3-(3,4-Bis-hydroxyméthyl-benzyloxy)-phényl]-3-éthyl-7-méthyl-octan-3-ol.

En particulier, le principe actif pharmaceutique incorporé dans la composition selon l'invention est le (4E,6E)-7-[3-(3,4-Bis-hydroxyméthyl-benzyloxy)-phényl]-3-éthyl-nona-4,6-dien-3-ol .

### La composition de l'invention :

La composition de l'invention est de préférence sous la forme d'une émulsion.

On entend par « émulsion », au sens de la présente description, une composition comprenant au moins deux phases de polarités distinctes, dans laquelle au moins une phase est dispersée dans une autre phase.

Les compositions concernées par la présente invention sont les émulsions « simples » telles que eau dans huile (E/H), huile dans eau (H/E), eau dans silicone, glycol dans silicone, ou les gels-crèmes (phase huileuse dispersée dans une phase aqueuse par un émulsionnant polymérique non-tensioactif) ou les émulsions triples (par exemple E/H/E, H/E/H).

La composition selon l'invention est de préférence une émulsion présentant au moins une phase hydrophile et au moins une phase lipophile, ou encore au moins une phase glycolique ou hydroglycolique et au moins une phase lipophile.

Les proportions de chacune des phases sont choisies en fonction du principe actif et de sa solubilité dans la phase hydrophile et dans la phase lipophile, et ceci afin d'atteindre la concentration en principe actif thérapeutiquement efficace, pour la ou les pathologies visées.

Selon l'invention, la phase hydrophile représente 10 à 90 % (exprimé en poids par rapport au poids total de la composition), de préférence 10 à 45 % de la composition.

Selon l'invention, la phase lipophile représente 10 à 90 % (exprimé en poids par rapport au poids total de la composition), de préférence 15 à 50% en poids de la composition.

### Phase hydrophile de la composition :

Les composés de la phase hydrophile sont choisis en fonction du profil de solubilité du principe actif que l'on veut y solubiliser.

La phase hydrophile comprend au moins un solvant hydrophile, qui peut être choisi notamment parmi l'eau ou un mélange d'eau et d'alcool(s) en C1 à C4, tels que l'éthanol, l'isopropanol ou le butanol (solution hydroalcoolique), ainsi que les glycols ou bien leurs mélanges, notamment les mélanges glycols/eau (phase hydroglycolique). De plus, il est à noter que le solvant hydrophile peut être exclusivement glycolique. Un exemple d'émulsions utilisant une telle phase hydrophile est une émulsion de type Glycol/Silicone.

De préférence, au moins une phase hydrophile de la composition selon l'invention est une phase glycolique ou hydroglycolique.

De préférence, au moins une phase hydrophile de la composition selon l'invention comprend le principe actif solubilisé dans au moins un solvant hydrophile, et au moins un autre composé hydrophile. Cet autre composé peut être un autre solvant hydrophile, ou tout autre composé introduit dans la phase hydrophile.

Les glycols utilisables dans la présente invention incluent des alkylènes ou des polyalkylènes glycols. A titre d'exemples non limitatifs, on peut citer les alkylènes et polyalkylènes glycols (C1 à C6) tel que l'éthylène glycol, les polyéthylène glycols (2 à 20 monomères), le propylène glycol, le dipropylène glycol, le butylène glycol, le pentylène glycol, l'hexylène glycol.

De préférence, les glycols utilisés selon l'invention sont choisis parmi le propylèneglycol ou le PEG 400.

### Phase lipophile de la composition:

Les composés de la phase lipophile sont également choisis en fonction du profil de solubilité du principe actif que l'on veut y solubiliser.

Par phase lipophile, on entend une phase contenant, d'une part le principe actif, et d'autre part des composés exclusivement lipophiles. Ainsi, une telle phase ne comprend aucun composé hydrophile.

La phase lipophile comprend au moins un solvant lipophile, qui peut être une huile, éventuellement volatile. De préférence, elle comprend au moins une huile (éventuellement volatile) solvante de la molécule et/ou une cire.

De préférence, au moins une phase lipophile de la composition selon l'invention comprend le principe actif solubilisé dans au moins un solvant lipophile, et au moins un autre composé lipophile. Cet autre composé peut être un autre solvant lipophile, ou tout autre composé introduit dans la phase lipophile.

Selon l'invention, la cire peut être une cire animale, végétale, minérale ou synthétique.

Parmi les cires animales, on peut notamment citer les cires d'abeilles, la cire de baleine. Parmi les cires végétales, on peut citer, entre autres, les cires de Carnauba, de Candellila, d'Ouricoury, les cires de fibres de liège, les cires de canne à sucre et les cires du Japon. Parmi les cires minérales, on peut citer, en particulier, les cires de paraffine, les cires microcristallines, les cires de lignite et les ozokérites. Enfin, parmi les cires synthétiques, on peut citer, notamment, les cires de polyéthylène et les cires obtenues par synthèse de Fisher et Tropsch.

On peut citer parmi les huiles (solvant lipophile) utilisables dans la présente invention, l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide; des huiles d'hydrocarbures telles que des huiles de paraffine, le squalane, la vaseline; des esters tels que le myristate d'isopropyle, le palmitate d'isopropyle, le stéarate de butyle, le laurate d'hexyle, l'isononanoate d'isononyle, l'isononaoate de cétéaryle, le palmitate de 2-éthyl hexyle, le laurate de 2-hexyl décyle, le palmitate de 2-octyl décyle, le myristate de 2-octyl dodécyle, le succinate de 2-diéthylhexyle, le malate de diisostéaryle, le lactate de 2-octyl dodécyle, le triisostéarate de glycérine, le triglycéride caprylique / caprique, le benzoate d'alkyle en C₁₂₋₁₅, des huiles perfluorées et/ou organofluorées; des acides gras supérieurs tels que l'acide myristique, l'acide palmitique, l'acide stéarique, l'acide béhénique, l'acide oléique, l'acide linoléique, l'acide linolénique, l'acide isostéarique, des alcools gras supérieurs tels que le cétanol, l'alcool stéarylique, l'alcool oléique, les alcools de Guerbet comme l'hexyl décanol, l'octyl dodécanol. Les huiles utilisables selon l'invention peuvent également être des huiles siliconées de type siloxanes linéaires et plus préférentiellement l'hexaméthyldisiloxane. On peut citer à titre d'exemple le produit commercialisé par la société DOW CORNING, le DC Fluid ^{®} 0.65cSt.

Les composants de la phase lipophile selon l'invention sont préférentiellement choisis parmi le palmitate d'isopropyle, le triglycéride caprylique /caprique ( Miglyol 812 ), le benzoate d' alkyle en C12-C15 ( Finsolv TN ), l'isononanoate de cétéaryle ( Cetiol SN ), l'octyl -dodécanol ( Eutanol G ).

Des tensio-actifs sont également avantageusement utilisés pour obtenir une émulsion stable. Ils sont également de préférence utilisés pour obtenir des émulsions très fines, notamment dans le cas des émulsions multiples.

Les tensio-actifs (émulsionnants ou surfactants) sont des substances naturelles ou synthétiques formées d'une partie hydrophile ou polaire et d'une partie lipophile ou apolaire. Ce sont des molécules amphiphiles puisqu'elles ont une double polarité. Les tensio-actifs sont caractérisés par leur HLB. Les tensio-actifs se mettent à l'interface des phases hydrophile et lipophile. La valeur du HLB du tensio-actif oriente le sens de l'émulsion.

De préférence, la composition sous forme d'émulsion selon l'invention comprend au moins un tensioactif pharmacologiquement ou cosmétologiquement acceptable n'interagissant pas avec le principe actif, présent de préférence dans la phase continue de l'émulsion.

Ainsi, un ou plusieurs tensio-actif(s) de haut HLB (15-16 par exemple) sont incorporés dans la phase hydrophile de l'émulsion afin de préparer une émulsion H / E et peuvent contribuer à la solubilisation du principe actif incorporé dans la phase hydrophile.

De même, un ou plusieurs tensio-actifs de bas HLB (5-6) sont incorporés dans la phase lipophile de l'émulsion afin de préparer une émulsion E / H et peuvent contribuer à la solubilisation du principe actif dans la phase lipophile.

Les tensio-actifs utilisés dans la présente invention doivent être pharmacologiquement ou cosmétologiquement acceptables et ne doivent pas interagir avec le principe actif. Généralement, les tensio-actifs sont incorporés avant émulsification dans la phase destinée à former la phase continue de l'émulsion.

A titre d'exemples non limitatifs de tensio-actifs non ioniques de haut HLB, on peut citer les esters de glycérol, les esters de polyoxyethylèneglycol ou polyoxypropyléneglycol, les esters de saccharose et d'acides gras, les esters de sorbitanne polyoxyethylènés, les tensio-actifs à liaison ether oxyde comme les alcools polyoxyethylénés ou polyoxypropylénés.

De préférence, pour la réalisation d'émulsions de type huile dans eau selon l'invention, on utilise les esters de polyoxyethylèneglycol comme le PEG 100-stéarate (Arlacel^{®} 165) ou les alcools polyoxyethylénés comme le Ceteareth-20 (Eumulgin^{®} B2).

A titre d'exemples non limitatifs de tensio-actifs non ioniques de bas HLB, on peut citer les esters de glycols, les esters de sorbitanne, les alcools polyoxyethylénés.

De préférence, pour la réalisation d'émulsions de type eau dans huile selon l'invention, on utilise les esters de sorbitanne comme le laurate de sorbitanne, l'oléate de sorbitanne, le stéarate de sorbitanne.

A titre d'exemples non limitatifs de tensioactifs siliconés permettant de formuler des émulsions siliconées, notamment eau / silicone ou glycol / silicone, on peut citer les tensioactifs :
- de type polyalkyles méthicone copolyols (polyalkyles méthylsiloxane oxyalkylénés éventuellement réticulés) contenant des chaînes alkyles de C6 à C20, saturées ou non, linéaires ou ramifiées, un motif polyoxyéthyléné de 1 à 50 OE (oxyde d'éthylène) et/ou un motif polyoxypropyléné de 1 à 50 OP (oxyde de propylène) et
- de type polyalkyles diméthyles méthylsiloxane oxyalkylénés contenant : des chaînes alkyles de C6 à C20, saturées ou non, linéaires ou ramifiées, un motif polyoxyéthyléné de 1 à 50 OE et/ou un motif polyoxypropyléné de 1 à 50 OP.

Avantageusement, on utilisera donc comme émulsionnants siliconés, les alkyldiméthicone copolyols tels que l' Abil EM-90, ou le mélange de diméthicone copolyol et cyclométhicone, vendu par la société Dow Corning sous la dénomination 3235C Formulation Aid, le laurylmethicone copolyol vendu sous le nom d'Emulsifier 10 par Dow Corning, ou des mélanges à base d'un polymère siliconé tel que le cetyl dimethicone copolyol avec du polyglyceryl-4 isostéarate et de l'hexyl laurate vendu sous le nom d'Abil WE09 par la société Goldschmidt , - Abil EM 97 de Goldschmidt (Dimethicone copolyol & cyclomethicone), le Wacker SPG 128 VP de Wacker (cyclomethicone et octyldimethicone methoxy glycosyl) , le Silwax WD-IS (Dimethicone copolyol iso-stearate). On pourra également utiliser les émulsionnants siliconés non polymériques comme les mono ou polyalkylesters siloxanes, par exemple le Silwax S de Lambent (Dimethiconol stearate),ou des esters d'acide alkoxylés carboxyliques comme les alkylesters polyhydroxylés de PEG, par exemple l'Arlacel P 135 de Uniqema (PEG-30 d ipolyhydroxystearate

La formulation des émulsions au sein d'une composition pharmaceutique ou cosmétique fait appel aux techniques usuelles de l'homme du métier, dans le domaine de la formulation et de la galénique.

La composition selon l'invention est de préférence adaptée à une application topique sur la peau, les phanères et/ou les muqueuses. La composition comprend une quantité de principe actif suffisante pour obtenir l'effet recherché.

D'autres composés peuvent être ajoutés pour moduler la pénétration cutanée, que ce soit pour l'améliorer ou la diminuer.

Parmi ces composés additionnels modulant la pénétration cutanée du principe actif, on peut citer par exemple les promoteurs d'absorption hydrophiles, les promoteurs d'absorption lipophiles et les agents limitant la pénétration cutanée en piégeant la molécule.

Parmi les promoteurs d'absorption hydrophiles, on peut citer par exemple le Propylèneglycol, le Transcutol, le DMSO, l'éthanol, la N-methyl pyrrolidone, et de préférence le Propylèneglycol.

Parmi les promoteurs d'absorption lipophiles, on peut citer par exemple l'acide oléique, l'oleyl alcool, l'oleyl oléate, l'oleyl érucate, l'huile de macadamia ou l'alpha-bisabolol, les terpènes, l'azone, et de préférence l'acide oléique.

Parmi les agents limitant la pénétration cutanée du principe actif, on peut citer par exemple les polyéthyleneglycols, et de préférence le PEG 400. Ces composés, par leur structure en réseau, sont connus pour piéger certaines molécules dans ce réseau et freiner leur libération du réseau vers le tissu cible.

La présente invention peut comprendre en outre tout additif usuellement utilisé dans le domaine cosmétique ou pharmaceutique, tel que des agents séquestrants, des agents mouillants, des agents d'adhérence, des agents d'étalement, des antioxydants, des filtres solaires, des conservateurs, des charges, des électrolytes, des humectants, des pigments, des colorants, des bases ou des acides usuels, minéraux ou organiques, des huiles essentielles, des actifs cosmétiques, des hydratants, des vitamines, des acides gras essentiels et/ou des sphingolipides.

Bien entendu, ce ou ces éventuels composés complémentaires, et/ou leur quantité, sont choisis de manière telle que les propriétés avantageuses de l'émulsion ne soient pas, ou substantiellement pas, altérées.

### Fabrication de la composition :

L'invention a également pour objet un procédé de fabrication d'une composition cosmétique ou pharmaceutique telle que décrite précédemment.

On distingue cependant deux procédés différents en fonction de la sensibilité du principe actif à la température. Par non sensible à la température, on entend un principe actif supportant des températures entre 40 et 80°C sans subir de dégradation physique et/ou chimique.

### Procédé A :

Dans le cas d'un principe actif non sensible à la température, il peut être chauffé avec les autres constituants des différentes phases. Les étapes du procédé sont les suivantes :
i) mélange dudit principe actif avec au moins un solvant hydrophile à une température comprise entre 40 et 80°C, afin d'obtenir une phase hydrophile dans laquelle le principe actif est présent sous forme solubilisée ;
ii) mélange dudit principe actif avec au moins un solvant lipophile à une température comprise entre 40 et 80°C, afin d'obtenir une phase lipophile dans laquelle le principe actif est présent sous forme solubilisée ;
iii) mélange de la phase hydrophile obtenue en i) avec la phase lipophile obtenue en ii).

De préférence, le procédé comprend en fin d'étape ii) l'ajout d'au moins un autre composé lipophile dans ladite phase lipophile.

Ledit procédé comprend ainsi dans ce cas les étapes suivantes :
i) mélange dudit principe actif avec au moins un solvant hydrophile à une température comprise entre 40 et 80°C, afin d'obtenir une phase hydrophile dans laquelle le principe actif est présent sous forme solubilisée ;
ii) mélange dudit principe actif avec au moins un solvant lipophile à une température comprise entre 40 et 80°C, afin d'obtenir une phase active lipophile dans laquelle le principe actif est présent sous forme solubilisée, puis mélange de cette phase active lipophile avec au moins un autre composé lipophile, afin d'obtenir une phase lipophile ;
iii) mélange d'au moins une phase hydrophile obtenue en i) avec au moins une phase lipophile obtenue en ii).

Avantageusement, le procédé selon l'invention comprend l'ajout d'au moins un agent tensio-actif de HLB adapté au cours de l'étape i) ou de l'étape ii). Un agent tensio-actif de haut HLB sera introduit dans la phase hydrophile, tandis qu'un agent tensio-actif de bas HLB sera introduit dans la phase lipophile.

Ainsi, de façon préférentielle, le procédé de préparation de la composition selon l'invention comprend les étapes suivantes :

### 1 - Préparation de la phase hydrophile :

a)- Peser l'eau et chauffer de préférence entre 60 et 80°C sous faible agitation.
b)- Ajouter le principe actif, le conservateur, l'agent chélatant et l'ajusteur de pH et le cas échéant le tensio-actif de haut HLB et agiter jusqu'à solubilisation complète de ces composés.
c)-Incorporer l'agent épaississant et agiter de préférence de 20 à 40 minutes à une température comprise entre 60 et 80°C, de préférence à la température de 75°C.
d)-Ajouter ensuite le ou les agents stabilisant(s) de l'émulsion et laisser sous agitation jusqu'à complète solubilisation.

### 2 - Préparation de la phase lipophile :

a)- Peser l'ensemble des constituants de la phase lipophile (excepté le principe actif) et, dans le cas où le tensio-actif de haut HLB n'est pas incorporé dans la phase hydrophile, le tensio-actif de bas HLB, et chauffer de préférence entre 60 et 80°C sous agitation, jusqu'à l'obtention d'une phase homogène.
b)-Le principe actif, dans sa proportion destiné à cette phase, est ensuite ajouté à la phase lipophile laissée sous agitation jusqu'à sa solubilisation complète.
   - Dans un mode préféré du procédé selon l'invention, le principe actif est solubilisé préalablement à la réalisation de la phase a) ci-dessus dans un solvant lipophile du principe actif, afin d'obtenir un mélange comprenant le principe actif. L'étape b) consiste alors à ajouter ce mélange comprenant le principe actif dans sa proportion destiné à cette phase, à la phase lipophile laissée sous agitation jusqu'à sa solubilisation complète.

### 3 - Emulsification :

- Verser la phase hydrophile dans la phase lipophile et émulsionner sous agitation forte pendant un temps compris entre 5 et 20 minutes de préférence pendant 10 minutes.

### Procédé B :

Dans le cas d'un principe actif sensible à la température, le procédé devra être modifié pour éviter au maximum de chauffer le principe actif à des températures supérieures à 40°C. Lorsque des étapes de chauffage sont nécessaires par exemple afin de rendre liquides des constituants solides à température ambiante, alors le temps de contact du principe actif avec la phase à température supérieure à 40°C sera réduit au minimum.

Les étapes du procédé sont les suivantes :
i) mélange dudit principe actif avec au moins un solvant hydrophile à une température inférieure à 40°C, afin d'obtenir une phase active hydrophile dans laquelle le principe actif est présent sous forme solubilisée ;
ii) préparation d'une phase non active hydrophile par chauffage d'au moins un autre composé hydrophile à une température comprise entre 40 et 80°C ;
iii) mélange dudit principe actif avec au moins un solvant lipophile à une température inférieure à 40°C, afin d'obtenir une phase lipophile dans laquelle le principe actif est présent sous forme solubilisée ;
iv) mélange de la phase non active hydrophile obtenue en ii) avec la phase lipophile obtenue en iii) à une température d'environ 50 à 60°C, afin d'obtenir une émulsion ;
v) refroidissement de l'émulsion obtenue en iv), jusqu'à une température de 30 à 45°C ;
vi) mélange de ladite émulsion obtenue en v) avec ladite phase active hydrophile obtenue en i), à une température comprise entre 30 et 45°C .

Avantageusement, le procédé selon l'invention comprend l'ajout d'au moins un agent tensio-actif de HLB adapté au cours de l'étape ii) (préparation de la phase non active hydrophile) ou de l'étape iii) (préparation de la phase lipophile).

De préférence, le procédé comprend les étapes suivantes :
i) mélange dudit principe actif avec au moins un solvant hydrophile à une température inférieure à 40°C, afin d'obtenir une phase active hydrophile dans laquelle le principe actif est présent sous forme solubilisée ;
ii) préparation d'une phase non active hydrophile par chauffage d'au moins un autre composé hydrophile à une température comprise entre 40 et 80°C ;
iii) mélange dudit principe actif avec au moins un solvant lipophile à une température inférieure à 40°C, afin d'obtenir une phase active lipophile dans laquelle le principe actif est présent sous forme solubilisée ;
iv) préparation d'une phase non active lipophile par chauffage d'au moins un autre composé lipophile à une température comprise entre 40 et 80°C ;
v) refroidissement de la phase non active lipophile obtenue en iv) jusqu'à une température d'au plus 40°C ;
vi) mélange de la phase non active lipophile refroidie obtenue en v) avec la phase active lipophile obtenue en iii) ;
vii) mélange de la phase non active hydrophile obtenue en ii) avec la phase lipophile obtenue en vi) à une température d'environ 50 à 60°C, afin d'obtenir une émulsion ;
viii) refroidissement de l'émulsion obtenue en vii), jusqu'à une température de 30 à 45°C ;
ix) mélange de ladite émulsion obtenue en viii) avec ladite phase active hydrophile obtenue en i), à une température comprise entre 30 et 45°C .

Avantageusement, le procédé selon l'invention comprend l'ajout d'au moins un agent tensio-actif de HLB adapté au cours de l'étape ii) (préparation de la phase non active hydrophile) ou de l'étape iv) (préparation de la phase non active lipophile). Un agent tensio-actif de haut HLB sera introduit dans la phase hydrophile, tandis qu'un agent tensio-ctif de bas HLB sera introduit dans la phase lipophile.

Ainsi, de façon préférentielle, le procédé de préparation de la composition selon l'invention comprend les étapes suivantes :

### 1 - Préparation de la phase hydrophile :

a)-Peser l'eau et chauffer de préférence entre 60 et 80°C sous faible agitation.
b)-Ajouter le conservateur, l'agent chélatant et l'ajusteur de pH et les cas échéant, le tensio-actif de haut HLB, et agiter jusqu'à solubilisation complète de ces composés.
c)-Incorporer l'agent agent épaississant et agiter de préférence de 20 à 40 minutes à une température comprise entre 60 et 80°C, de préférence à la température de 75°C.
d)-Ajouter ensuite le ou les agents stabilisant(s) de l'émulsion et laisser sous agitation jusqu'à complète solubilisation.
e)-Par ailleurs, solubiliser la proportion de principe actif destinée à la phase hydrophile dans un mélange glycol/anti-oxydant, à une température inférieure à 40°C, de préférence à température ambiante.

### 2 - Préparation de la phase lipophile :

a)-Identifier les constituants de la phase lipophile qui sont liquides à température ambiante et ceux solides qui nécessitent d'être fondus.
b)-Identifier au moins un des constituants de la phase lipophile liquide à température ambiante et solvant du principe actif et y solubiliser le principe actif.
c)-Peser l'ensemble des autres constituants de la phase lipophile et, dans le cas où le tensio-actif de haut HLB n'est pas incorporé dans la phase hydrophile, le tensio-actif de bas HLB, et chauffer de préférence à une température nécessaire pour fondre les constituants solides, à savoir à une température entre 60 et 80°C sous agitation, jusqu'à l'obtention d'une phase homogène.
   De préférence laisser la température de la phase refroidir au maximum avant l'étape suivante.
d)-Ajouter le mélange actif /ingrédient lipophile à cette phase lipophile laissée sous agitation jusqu'à l'obtention d'une nouvelle phase homogène en minimisant le temps de contact du principe actif avec la température potentiellement élevée de la phase lipophile. Procéder rapidement à l'émulsification et au refroidissement suivant.

### 3 - Emulsification :

a)-Verser la phase hydrophile obtenue en fin d'étape d) de la préparation de la phase hydrophile, dans la phase lipophile et émulsionner sous agitation forte pendant un temps compris entre 5 et 20 minutes de préférence pendant 10 minutes à une température d'environ 50 à 60°C.
b)-Refroidir lentement en maintenant l'émulsion sous agitation modérée.
c)-A une température comprise entre 30 et 45°C et de préférence vers 35-40°C, incorporer le principe actif destiné à la phase hydrophile et préalablement solubilisé dans le mélange glycol / antioxydant, (étape e) de la préparation de la phase hydrophile) dans l'émulsion et maintenir l'agitation jusqu'au retour de l'émulsion à température ambiante.

Dans ce qui précède, l'agitation des solutions ou des émulsions selon la présente description peut être réalisée au moyen d'appareils conventionnels, tels que par exemple un appareil Rayneri.

Par agitation faible selon l'invention, on entend une agitation obtenue à partir d'un appareil Rayneri, d'une valeur inférieure à 300trs/min.

Par agitation modérée selon l'invention, on entend une agitation obtenue à partir d'un appareil Rayneri, comprise entre 301 et 700 trs/min.

Par agitation forte selon l'invention, on entend une agitation obtenue à partir d'un appareil Rayneri, comprise entre 701 et 1500 trs/min.

Par température ambiante, on entend une température comprise entre 20 et 30°C, de préférence comprise entre 23 et 27°C, préférentiellement une température égale à 25°C.

Selon un mode de réalisation particulièrement préféré, la composition préparée selon le procédé de l'invention est une émulsion de type huile dans eau H/E, en d'autres termes une émulsion dans laquelle ladite première phase selon la description est une phase lipophile dispersée dans une deuxième phase continue, hydrophile.

### Applications cosmétiques ou pharmaceutiques :

Les compositions selon l'invention peuvent être utilisées pour une application pharmaceutique ou cosmétique. Elles se présentent de préférence sous une forme adaptée à usage topique, par exemple sous forme de crème, mousse, spray, aérosol ou sous toute forme d'administration compatible avec la formulation revendiquée.

De préférence, la composition selon l'invention contient de la vitamine D ou un dérivé de vitamine D et est applicable en dermatologie, par exemple dans les applications indiquées ci-après, de préférence dans le traitement du psoriasis.

Cette composition convient particulièrement bien :
1) pour traiter les affections dermatologiques liées à un désordre de la différenciation ou de la prolifération des kératinocytes ou des sébocytes notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) pour traiter des troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal) ;
3) pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ;
4) pour traiter certaines affections inflammatoires cutanées ne présentant pas de trouble de la kératinisation, telles que l'eczéma atopique et les allergies de contact ;
5) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires ;
6) pour traiter d'autres désordres dermatologiques tels que les dermatoses bulleuses et les maladies du collagène ;
7) pour prévenir ou traiter les signes du vieillissement de la peau, qu'il soit photo-induit ou chronologique ou pour réduire les pigmentations et les kératoses actiniques, ou toutes pathologies cutanées associées au vieillissement chronologique ou actinique ;
8) pour prévenir ou traiter les troubles de la cicatrisation ou pour prévenir ou réparer les vergetures ;
9) pour lutter contre les troubles de la fonction sébacée tels que l'hyperséborrhée de l'acné ou la séborrhée simple ou encore l'eczéma séborrhéique ;
10) pour traiter certains troubles ophtalmologiques, notamment les cornéopathies ;
11) pour le traitement ou la prévention des états cancéreux ou précancéreux de cancers cutanés ou non présentant ou pouvant être induits pour présenter des récepteurs de vitamine D, tels que, mais sans limitation, le cancer du sein, la leucémie, les syndromes myélodysplasiques et les lymphomes, les carcinomes des cellules de l'épithélium malpighien et les cancers gastro-intestinaux, les mélanomes, et l'ostéosarcome ;
12) pour le traitement d'affections inflammatoires telles que l'arthrite ou la polyarthrite rhumatoïde ;
13) pour le traitement de toute affection d'origine virale au niveau cutané ou général ;
14) pour la prévention ou le traitement de l'alopécie de différentes origines, notamment l'alopécie due à la chimiothérapie ou aux rayonnements ;
15) pour le traitement d'affections dermatologiques ou générales à composante immunologique ;
16) pour le traitement d'affections immunitaires, telles que les maladies auto-immunes (comme, mais sans limitation, le diabète sucré de type 1, la sclérose en plaques, le lupus et les affections de type lupus, l'asthme, la glomérulonéphrite, etc.), des dysfonctionnements sélectifs du système immunitaire (par exemple le SIDA) et la prévention du rejet immun, comme les rejets de greffons (par exemple le rein, le coeur, la moelle osseuse, le foie, des îlots pancréatiques ou tout le pancréas, la peau, etc.) ou la prévention de la maladie du greffon contre l'hôte ;
17) pour le traitement d'affections endocriniennes pouvant être traitées par des analogues de vitamine D tels que ceux modulant avantageusement la sécrétion hormonale, par exemple, par l'augmentation de la sécrétion d'insuline ou la suppression sélective de la sécrétion de l'hormone parathyroïdienne (par exemple dans l'insuffisance rénale chronique et l'hyperparathyroïdie secondaire) ;
18) pour le traitement d'affections caractérisées par une gestion anormale du calcium intracellulaire ; et
19) pour le traitement et ou la prévention des carences en vitamine D et d'autres affections de l'homéostasie des minéraux dans le plasma et les os, tel que le rachitisme, l'ostéomalacie, l'ostéoporose, notamment dans le cas des femmes monopausées, l'ostéodystrophie rénale, les troubles de la fonction parathyroïdienne.

Un objet particulier de l'invention est donc une émulsion comprenant une phase hydrophile et une phase lipophile, de la vitamine D ou un analogue ou un dérivé de vitamine D, à savoir de préférence le (4E,6E)-7-[3-(3,4-Bis-hydroxyméthyl-benzyloxy)-phényl]-3-éthyl-nona-4,6-dien-3-ol étant présent et sous forme solubilisée dans chacune de ces phases.

Un objet de l'invention concerne plus particulièrement une émulsion comprenant une phase glycolique ou hydroglycolique et une phase lipophile, du (4E,6E)-7-[3-(3,4-Bis-hydroxyméthyl-benzyloxy)-phényl]-3-éthyl-nona-4,6-dien-3-ol étant présent sous forme solubilisée dans chacune de ces phases.

Un autre objet de l'invention est une composition pharmaceutique ou cosmétique à usage topique comprenant une telle émulsion.

Un autre objet de l'invention est l'utilisation d'une composition telle que définie ci-dessus, notamment une émulsion, pour la préparation d'un médicament destiné au traitement des désordres cutanés, notamment le psoriasis.

En effet, la vitamine D et ses analogues ou dérivés limitent la production excessive de cellules cutanées sur les surfaces atteintes et possèdent des avantages avérés pour le traitement de cette affection qui se caractérise notamment par la présence de lésions épaisses, squameuses et sèches.

Les exemples ci-dessous permettent d'illustrer l'invention, sans toutefois en limiter la portée.

Dans les exemples 1 et 2, les quantités des constituants entrant dans la composition des formulations sont exprimées en pourcentage en poids par rapport au poids total de la composition.

### EXEMPLES

### Exemple 1 : Procédé de fabrication utilisable pour les exemples 2 ou 3)

### 1 - Préparation de la phase hydrophile :

- Peser l'eau et chauffer à 75°C, sous faible agitation.
- Ajouter l'EDTA, l'Hydrogénophosphate de sodium et le Methyl paraben et agiter de façon modérée jusqu'à solubilisation complète.

Incorporer le Veegum K et agiter de façon modérée 30 minutes, à 75°C.

Ajouter ensuite le Keltrol T et laisser sous agitation jusqu'à complète solubilisation.

### 2 - Préparation de la phase lipophile :

- Peser l'ensemble des constituants de la phase lipophile (excepté le (4E,6E)-7-[3-(3,4-bis-hydroxyméthyl-benzyloxy)-phényl]-3-éthyl-nona-4,6-dien-3-ol) et chauffer à 75 ° C sous agitation modérée, jusqu'à l'obtention d'une phase homogène.
- Ajouter le (4E,6E)-7-[3-(3,4-bis-hydroxyméthyl-benzyloxy)-phényl]-3-éthyl-nona-4,6-dien-3-ol destiné à cette phase et laisser sous agitation jusqu'à solubilisation complète.

### 3 - Emulsification :

- Verser la phase hydrophile dans la phase lipophile et émulsionner sous agitation forte (1200 trs / min) pendant 10 minutes à une température d'environ 60°C.
- Refroidir lentement en maintenant l'agitation modérée à 700 trs/min
- Vers 35-40°C, incorporer le (4E,6E)-7-[3-(3,4-bis-hydroxyméthyl-benzyloxy)-phényl]-3-éthyl-nona-4,6-dien-3-olpréalablement solubilisé dans le mélange glycol / BHA ,dans l'émulsion et maintenir l'agitation jusqu'à retour de l'émulsion à température ambiante.

### Exemple 2 : Emulsion H / E

| Ingrédients Dénomination INCI | Dénomination commerciale | Fonction dans l'émulsion | |
|---|---|---|---|
| **Phase LIPOPHILE** | | | % |
| Ceteareth-20 | Eumulgin B2 | Tensio-actif | 3.75 |
| Stearate de Glyceryle | Cutina GMS -V | Facteur de consistance | 6.25 |
| Triglyceride Caprylique / Caprique | Miglyol 812 | Emollient Solvant du principe actif | 15 |
| Palmitate d'isopropyle | Crodamol IPP | Emollient Solvant du principe actif | 10 |
| Vaseline | Vaseline Vara 5718 | Agent occlusif | 3 |
| Dimethicone | Silicone DC 200, 350 cs | Agent d'étalement | 1 |
| Propylparaben : Parahydroxybenzoate de Propyle | Propyl paraben | Conservateur | 0.05 |
| benzoate d'alkyle en C₁₂₋₁₅ | Tegosoft TN | Emollient Solvant du principe actif | 12 |
| Tocopherol | DL-alpha tocophérol | Agent antioxydant | 0.04 |
| (4,6E)-7-[3-(3,4-bis-hydroxyméthyl-benzyloxy)-phényl]-3-éthyl-nona-4,6-dien-3-ol | 4E,6E)-7-[3-(3,4-bis-hydroxyméthyl -benzyloxy)-phényl]-3-éthyl-nona-4,6-dien-3-ol | Principe actif | 0.1 |

| **Phase HYDROPHILE** | | | |
|---|---|---|---|
| Gomme Xanthane | Keltrol T | Epaississant / Stabilisant | 0.22 |
| Methylparaben | Methyl paraben | Conservateur | 0.2 |
| Silicate de Magnesium Aluminium | Veegum K | Epaississant / Stabilisant | 0.8 |
| Poly éthylène glycol 400 | PEG 400 | Solvant du principe actif | 20 |
| BHA Butylhydroxyanisole | BHA | Agent antioxydant | 0.01 |
| Disodium EDTA | EDTA 2Na | Agent chélatant | 0.1 |
| Disodiumhydrogenophoshate, dihydrate | Na₂HPO₄, 2H₂O | Ajusteur de pH | 0.4 |
| Eau purifiée | Eau purifiée | Solvant | qs100 |
| (4E,6E)-7-[3-(3,4-bis-hydroxyméthyl-benzyloxy)-phényl]-3-éthyl-nona-4,6-dien-3-ol | (4E,6E)-7-[3-(3,4-bis-hydroxyméthyl -benzyloxy)-phényl]-3-éthyl-nona-4,6-dien-3-ol | Principe actif | 0.2 |

### Exemple 3 : Emulsion H/E

| **Ingrédients** **Dénomination INCl** | Dénomination commerciale | Fonction dans l'émulsion | % |
|---|---|---|---|
| **Phase LIPOPHILE** | | | % |
| Ceteareth-20 | Eumulgin B2 | Tensio-actif | 3.75 |
| Stearate de Glyceryle | Cutina GMS - V | Facteur de consistance | 6.25 |
| Triglyceride Caprylique / Caprique | Miglyol 812 | Emollient Solvant du principe actif | 15 |
| palmitate d'isopropyle | Crodamol IPP | Emollient Solvant du principe actif | 10 |
| Vaseline | Vaseline Vara 5718 | Agent occlusif | 3 |
| Dimethicone | Silicone DC 200, 350 cs | Agent d'étalement | 1 |
| Propylparaben : Parahydroxybenzoate de Propyle | Propyl paraben | Conservateur | 0.05 |
| benzoate d'alkyle en C₁₂₋₁₅ | Tegosoft TN | Emollient Solvant du principe actif | 12 |
| BHT : Butylhydroxytoluène | BHT | Agent antioxydant | 0.04 |
| (4E,6E)-7-[3-(3,4-bis-hydroxyméthyl-benzyloxy)-phényl]-3-éthyl-nona-4,6-dien-3-ol | (4E,6E)-7-[3-(3,4-bis-hydroxyméthyl-benzyloxy)-phényl]-3-éthyl-nona-4,6-dien-3-ol | Principe actif | 0.2 |

| **Phase HYDROPHILE** | | | |
|---|---|---|---|
| Gomme Xanthane | Keltrol T | Epaississant / Stabilisant | 0.22 |
| Méthylparaben : Parahydroxybenzoate de Méthyle | Methyl paraben | Conservateur | 0.2 |
| Silicate de Magnesium Aluminium | Veegum K | Epaississant / Stabilisant | 0.8 |
| Propylene Glycol | Propylèneglycol | Solvant du principe actif | 20 |
| BHA Butylhydroxyanisole | BHA | Agent antioxydant | 0.01 |
| Hydroxypropyl methylcellulose | Methocel | Epaississant / Stabilisant | 0.2 |
| Purified water | Eau purifiée | Solvant | qs100 |
| (4E,6E)-7-[3-(3,4-bis-hydroxyméthyl-benzyloxy)-phényl]-3-éthyl-nona-4,6-dien-3-ol | (4E,6E)-7-[3-(3,4-bis-hydroxyméthyl-benzyloxy)-phényl]-3-éthyl-nona-4,6-dien-3-ol | Principe actif | 0.1 |

Les exemples cités ci-dessus illustrent l'intérêt de la solubilisation mixte.

En effet, la préparation d'une émulsion à 0.3 % de (4E,6E)-7-[3-(3,4-bis-hydroxyméthyl-benzyloxy)-phényl]-3-éthyl-nona-4,6-dien-3-ol a été rendue possible uniquement grâce à une solubilisation mixte de la molécule : une fraction dans la phase hydrophile et une fraction dans la phase lipophile.

Des essais de solubilisation du (4E,6E)-7-[3-(3,4-bis-hydroxyméthyl-benzyloxy)-phényl]-3-éthyl-nona-4,6-dien-3-ol uniphase (dans la phase hydrophile ou dans la phase lipophile) n'ont pas permis d'obtenir une émulsion physiquement stable dans le temps ; des phénomènes de croissance cristalline du (4E,6E)-7-[3-(3,4-bis-hydroxyméthyl-benzyloxy)-phényl]-3-éthyl-nona-4,6-dien-3-ol sont apparus dès le premier mois de stockage à 4°C.

En revanche, la solubilisation mixte a permis une bonne stabilité physique de l'émulsion et l'absence de croissance cristalline après 3 mois de stockage à 4°C.

## Revendications

1. Composition pharmaceutique ou cosmétique comprenant :
- au moins une phase hydrophile,
- au moins une phase lipophile, comprenant au moins un solvant lipophile,
- et au moins un principe actif,
**caractérisée en ce que** le logarithme du coefficient de partage du principe actif est compris entre 2 et 6, et **en ce que** le principe actif est un dérivé de la vitamine D et est présent sous forme solubilisée dans au moins une phase hydrophile et dans au moins le solvant lipophile de la phase lipophile.

2. Composition selon la revendication 1, **caractérisée en ce que** ladite phase hydrophile comprend le principe actif solubilisé dans au moins un solvant hydrophile, et au moins un autre composé hydrophile.

3. Composition selon l'une des revendications 1 ou 2, **caractérisée en ce que** la composition est une émulsion.

4. Composition selon l'une des revendications 1 à 3, comprenant une phase hydrophile et une phase lipophile.

5. Composition selon l'une quelconque des revendications 1 à 4, **caractérisée en ce qu'**au moins une phase hydrophile est une phase glycolique ou hydroglycolique.

6. Composition selon l'une des revendications 1 à 5, dans laquelle le principe actif est le (4E,6E)-7-[3-(3,4-Bis-hydroxyméthyl-benzyloxy)-phényl]-3-éthyl-nona-4,6-d ien-3-ol .

7. Composition selon l'une des revendications 1 à 6, comprenant en outre au moins un tensio-actif pharmacologiquement ou cosmétologiquement acceptable.

8. Composition selon l'une des revendications précédentes, qui est sous une forme adaptée à usage topique.

9. Utilisation d'une composition selon l'une des revendications 1 à 8 pour la préparation d'un médicament destiné au traitement du psoriasis.

10. Procédé de fabrication d'une composition telle que définie dans les revendications 1 à 8, **caractérisé en ce qu'**il comprend les étapes suivantes :
i) mélange dudit principe actif avec au moins un solvant hydrophile à une température comprise entre 40 et 80°C, afin d'obtenir une phase hydrophile dans laquelle le principe actif est présent sous forme solubilisée ;
ii) mélange dudit principe actif avec au moins un solvant lipophile à une température comprise entre 40 et 80°C, afin d'obtenir une phase lipophile dans laquelle le principe actif est présent sous forme solubilisée ;
iii) mélange de la phase hydrophile obtenue en i) avec la phase lipophile obtenue en ii).

11. Procédé selon la revendication 10, **caractérisé en ce qu'**il comprend en fin d'étape ii) l'ajout d'au moins un autre composé lipophile dans ladite phase lipophile.

12. Procédé selon la revendication 10 ou 11, **caractérisé en ce qu'**au moins un agent tensio-actif de HLB adapté est ajouté au cours de l'étape i) ou de l'étape ii).

13. Procédé de fabrication d'une composition telle que définie dans les revendications 2 à 8, **caractérisé en ce qu'**il comprend les étapes suivantes :
i) mélange dudit principe actif avec au moins un solvant hydrophile à une température inférieure à 40°C, afin d'obtenir une phase active hydrophile dans laquelle le principe actif est présent sous forme solubilisée ;
ii) préparation d'une phase non active hydrophile par chauffage d'au moins un autre composé hydrophile à une température comprise entre 40 et 80°C ;
iii) mélange dudit principe actif avec au moins un solvant lipophile à une température inférieure à 40°C, afin d'obtenir une phase lipophile dans laquelle le principe actif est présent sous forme solubilisée ;
iv) mélange de la phase non active hydrophile obtenue en ii) avec la phase lipophile obtenue en iii) à une température d'environ 50 à 60°C, afin d'obtenir une émulsion ;
v) refroidissement de l'émulsion obtenue en iv), jusqu'à une température de 30 à 45°C ;
vi) mélange de ladite émulsion obtenue en v) avec ladite phase active hydrophile obtenue en i), à une température comprise entre 30 et 45°C .

14. Procédé de fabrication d'une composition telle que définie dans les revendications 1 à 8, **caractérisé en ce qu'**il comprend les étapes suivantes :
i) mélange dudit principe actif avec au moins un solvant hydrophile à une température inférieure à 40°C, afin d'obtenir une phase active hydrophile dans laquelle le principe actif est présent sous forme solubilisée ;
ii) préparation d'une phase non active hydrophile par chauffage d'au moins un autre composé hydrophile à une température comprise entre 40 et 80°C ;
iii) mélange dudit principe actif avec au moins un solvant lipophile à une température inférieure à 40°C, afin d'obtenir une phase active lipophile dans laquelle le principe actif est présent sous forme solubilisée ;
iv) préparation d'une phase non active lipophile par chauffage d'au moins un autre composé lipophile à une température comprise entre 40 et 80°C ;
v) refroidissement de la phase non active lipophile obtenue en iv) jusqu'à une température d'au plus 40°C ;
vi) mélange d'au moins une phase non active lipophile refroidie obtenue en v) avec la phase active lipophile obtenue en iii) ;
vii) mélange de la phase non active hydrophile obtenue en ii) avec la phase lipophile obtenue en vi) à une température d'environ 50 à 60°C, afin d'obtenir une émulsion ;
viii) refroidissement de l'émulsion obtenue en vii), jusqu'à une température de 30 à 45°C ;
ix) mélange de ladite émulsion obtenue en viii) avec ladite phase active hydrophile obtenue en i), à une température comprise entre 30 et 45°C .

15. Procédé selon la revendication 14, **caractérisé en ce qu'**au moins un agent tensio-actif de HLB adapté est ajouté au cours de l'étape ii) ou de l'étape iv).
